# EUROPEAN PATENT APPLICATION

(11) **EP 4 534 545 A1**
(43) Date of publication of application: **09.04.2025**
(21) Application number: 23812219.6
(22) Date of filing: 26.05.2023
(51) Int. Cl.: C07K 7/06, A61K 38/00, A61P 25/28

(54) **NOVEL PEPTIDE AND USE THEREOF**

(30) Priority: 27.05.2022 KR 20220065264
(71) Applicant: Zincure Corp., Seoul 05006 (KR)
(72) Inventor: CHUNG, Sang Won, Seoul 06294 (KR); CHOUNG, Wonken, Seoul 06361 (KR); KIM, Ki-Ryeong, Seoul 02026 (KR); OH, Sang-Seok, Seoul 03021 (KR); LEE, Hyun Seung, Suwon-si Gyeonggi-do 16340 (KR)
(74) Representative: Dehns
(86) International application number: PCT/KR2023/007331
(87) International publication number: WO 2023/229445

(57) **Abstract**

The present invention relates to novel peptides and uses thereof, and more particularly to a novel peptide with a length of from 4 to 10 a.a. and a structure of H-G-X¹ -X² -G-X³, which have the ability to inhibit the formation of abnormal protein aggregates and autophagic activity against the protein aggregates.

## Description

### TECHNICAL FIELD

The present invention relates to novel peptides and uses thereof, and more particularly to novel peptides capable of inhibiting the formation of abnormal protein aggregates and removing the protein aggregates by autophagy, and to novel uses thereof in the treatment of neurodegenerative diseases.

### BACKGROUND ART

Zinc is a trace element that is essential for cell proliferation and differentiation and is known as a cofactor in the function and structure of proteins such as enzymes and transcription factors. In addition, the homeostasis of zinc in neurons is known to play an important role in the survival of neurons, and deficiency of zinc in neurons is known to induce apoptosis and cause neurodegenerative diseases such as Alzheimer's disease (AD) and Parkinson's disease (Lien, H. et al, BBRC. 268: 148-154, 2000). Conversely, excessive zinc in neurons are also known to induce cellular damage, which can lead to acute brain injury, such as focal ischemia and seizures (Koh et al., Science. 272: 1013-1016, 1996).

Autophagy is an intracellular mechanism that breaks down organelles during starvation to obtain energy or remove damaged organelles and abnormal or pathological protein aggregates. During autophagy, cytoplasmic components are surrounded by a bilayer membrane and isolated from other organelles to form an autophagosome. At this point, the soluble light chain I (LC3I) in the cytoplasm is converted to light chain II (LC3II), which is attached to the autophagosome membrane. The autophagosome then fuses with the lysosome to form an autolysosome, which is degraded and recycled by several hydrolytic enzymes present in the lysosome.

Recently, it has been shown that protein aggregates seen in neurodegenerative diseases, such as α-synuclein, β-amyloid, tau protein, superoxide dismutase-1 (SOD-1), Huntingtin protein, and TAR DNA-binding protein 43 (TDP-43), can be cleared by promoting autophagy. Indeed, there is evidence that defective autolysosomal function impairs the degradation of those protein aggregates, thereby blocking the flux of autophagic actions, and that this lack of autophagy ultimately leads to the accumulation of neurodegenerative disease byproducts (Zhang et al,. ABBS. 41(6): 437-445, 2009; Lee et al,. Cell. 141(7): 1146-1158, 2010).

Previous studies have shown that the function of lysosomes can be enhanced by zinc supply. On the other hand, it has been also reported that when the potent zinc chelator TPEN is applied to inhibit lysosomal membrane permeabilization (LMP) induced by H2O2, tamoxifen, and ethanol, and therefore the autophagic actions are reduced, additional zinc supply can promote autophagy (Lee et al, Glia 57: 1351-1361, 2009; Hwang et al., Biometals 23: 997-1013, 2010; Liuzzi et al., Biol. Trace Elem. Res. 156: 350-356, 2013; Kim et al., Front. Cell. Neurosci. 16: 895750, 2022). Hence, zinc is projected to play an important role in autophagy.

However, no drug has yet been developed to treat neurodegenerative diseases by maintaining zinc homeostasis within neurons.

### DETAILED DESCRIPTION OF THE INVENTION

### TECHNICAL PROBLEM

The present invention aims to address the above problems and others by providing novel peptides and their uses in the treatment of degenerative neurological diseases by maintaining zinc homeostasis in neurons, thereby removing abnormal or pathological protein aggregates such as amyloid β peptides, tau proteins, and superoxide dismutase, which are pathological substances in degenerative neurological diseases, through autophagy or enhanced lysosomal function.

### TECHNICAL SOLUTION

In one aspect of the present invention, there is provided a novel peptide with a length of from 4 to 10 a.a. and structural Formula 1 below, which has the ability to inhibit the formation of abnormal protein aggregates and autophagic activity against the protein aggregates.

H-G-X¹-X²-G-X³ (Structural formula 1)

(In the above structural formula, H is a L- or D-type histidine, G is glycine, X¹ is either absent or any D- or L-type amino acid, X² is any D- or L-type amino acid, and X³ is either absent or any D- or L-type amino acid except valine).

In another aspect of the present invention, there is provided a pharmaceutical composition for the treatment of neurodegenerative diseases comprising the peptide as an active ingredient.

In another aspect of the present invention, there is provided a method of treating a subject suffering from a neurodegenerative disease comprising administering a therapeutically effective amount of the peptide to the subject.

In another aspect of the present invention, there is provided a method of inhibiting the accumulation of a pathogenic protein aggregate in the nervous system of a subject suffering from a neurodegenerative disease, comprising administering a therapeutically effective amount of the peptide to the subject.

### EFFECT OF THE INVENTION

The novel peptides of the present invention, prepared as described above, can be utilized in the development of therapeutic agents to effectively treat neurodegenerative diseases such as Alzheimer's disease, Parkinson's disease, amyotrophic lateral sclerosis, Huntington's disease, etc. by preventing neuronal cell death and regulating intracellular zinc homeostasis. However, the scope of the present invention is not limited by these effects.

### BRIEF DESCRIPTION OF THE DRAWINGS

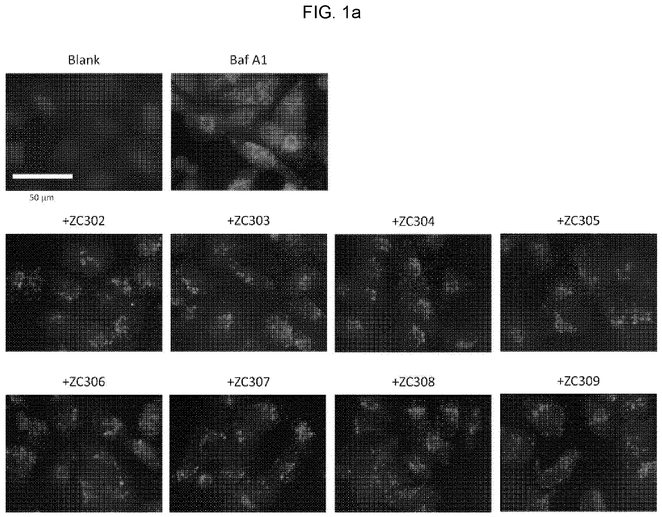

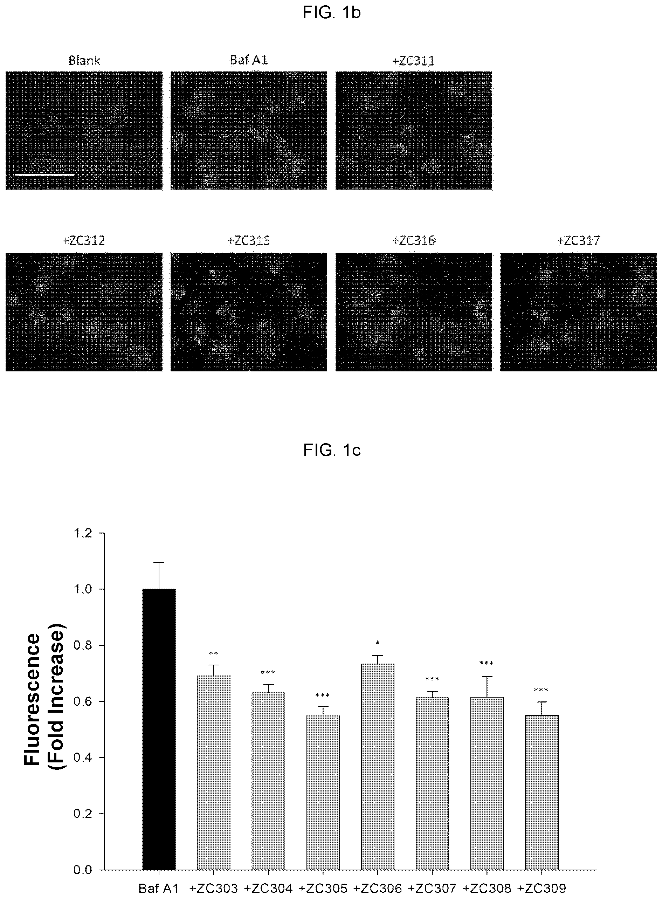

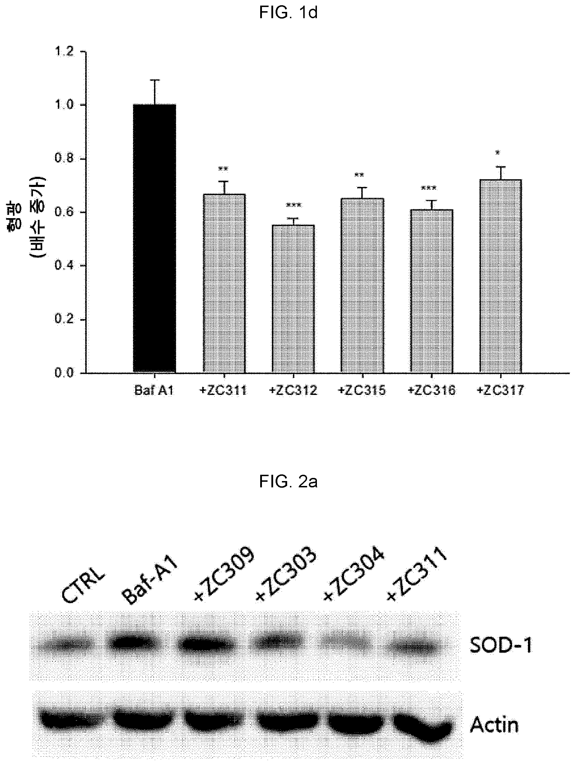

FIGS. 1a and 1b are a series of fluorescence micrographs showing that autophagic flow blocked by Bafilomycin A-1 is restored by the peptides according to one embodiment of the present invention, wherein an H4 cell line (GL-H4) stably overexpressing GFP-LC3 protein was treated with various peptides according to one embodiment of the present invention at a concentration of 20 µM in combination with Bafilomycin, whose LC3-GFP spots were photographed after 12 hours. FIGS. 1c and 1d are graphs showing quantification of the fluorescence intensity of LC3-GFP spots 12 hours after the treatment of the GL-H4 cell line with the peptides according to one embodiment of the present invention in combination with bafilomycin at a concentration of 20 µM. It can be seen that the spots have been significantly increased after the bafilomycin treatment due to the blocked autophagy, whereas the treatment with the peptides according to one embodiment of the present invention reduced the number and size of the spots, which is a result of the resolution of autophagy. In FIGS. 1C to 1D, * indicates a significant difference between the treatment with the peptide of one embodiment of the present invention and that with bafilomycin alone (* p < 0.05, ** p < 0.01 *** p < 0.001).

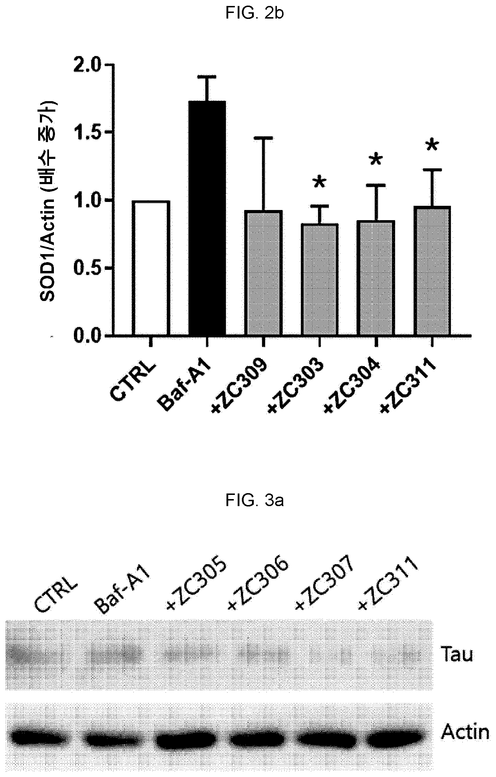

FIG. 2a is the results of a western blot analysis of SOD1 protein expression levels of protein samples obtained 18 h after treatment of HEK293T cells transfected to express SOD1 with either 100 nM bafilomycin alone or in combination with 30 µM of some peptides according to one embodiment of the present invention, to investigate the degradation of SOD1 protein aggregates by treatment with some peptides (ZC303, ZC304, ZC309, ZC311) according to one embodiment of the present invention. FIG. 2b is a graph showing the results of quantification of the relative expression. In FIG. 2b, * indicates a significant difference from the treatment with bafilomycin alone (** p* < 0.05).

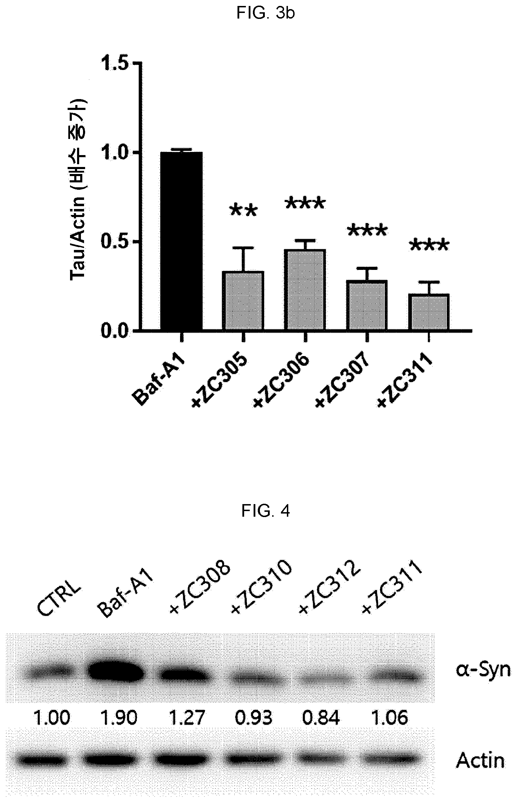

FIG. 3a is the results of a western blot analysis of mutant tau protein expression levels of protein samples obtained 18 h after treatment of HEK293T cells transfected to express mutant tau protein with either 100 nM bafilomycin alone or in combination with 30 µM of some peptides according to one embodiment of the present invention, to investigate the degradation of mutant tau protein aggregates by treatment with some peptides (ZC303, ZC304, ZC309, ZC311) according to one embodiment of the present invention. FIG. 3b is a graph showing the results of quantification of the relative expression. In FIG. 3b, * indicates a significant difference from the treatment with bafilomycin alone (** *p* < 0.01, **** p <* 0.001).
FIG. 4a is the results of a western blot analysis of α-synuclein expression levels of protein samples obtained 18 h after treatment of HEK293 T cells transfected to express α-synuclein protein with either 100 nM bafilomycin alone or in combination with 30 µM of some peptides according to one embodiment of the present invention, to investigate the degradation of α-synuclein protein aggregates by treatment with some peptides (ZC303, ZC304, ZC309, ZC311) according to one embodiment of the present invention.

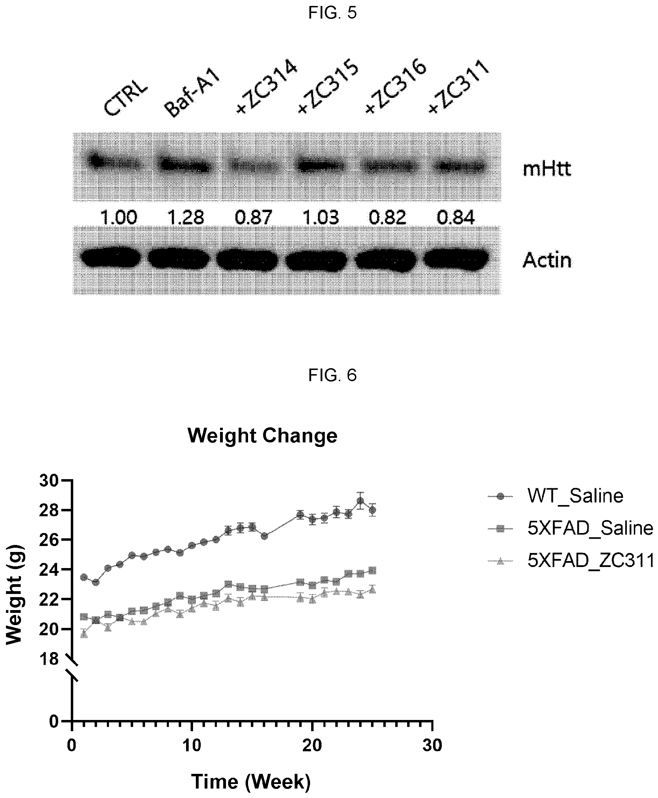

FIG. 5 is the results of a Western blot analysis of mutant huntingtin protein expression levels of protein samples obtained 18 h after treatment of HEK293T cells transfected to express mutant huntingtin protein with either 100 nM bafilomycin alone or in combination with 30 µM of some peptides according to one embodiment of the present invention, to investigate the degradation of mutant huntingtin protein aggregates by treatment with some peptides (ZC303, ZC304, ZC309, ZC311) according to one embodiment of the present invention.
FIG. 6 is a graph depicting the change in body weight over time after administration of the peptide according to one embodiment of the present invention to an Alzheimer's disease model animal.

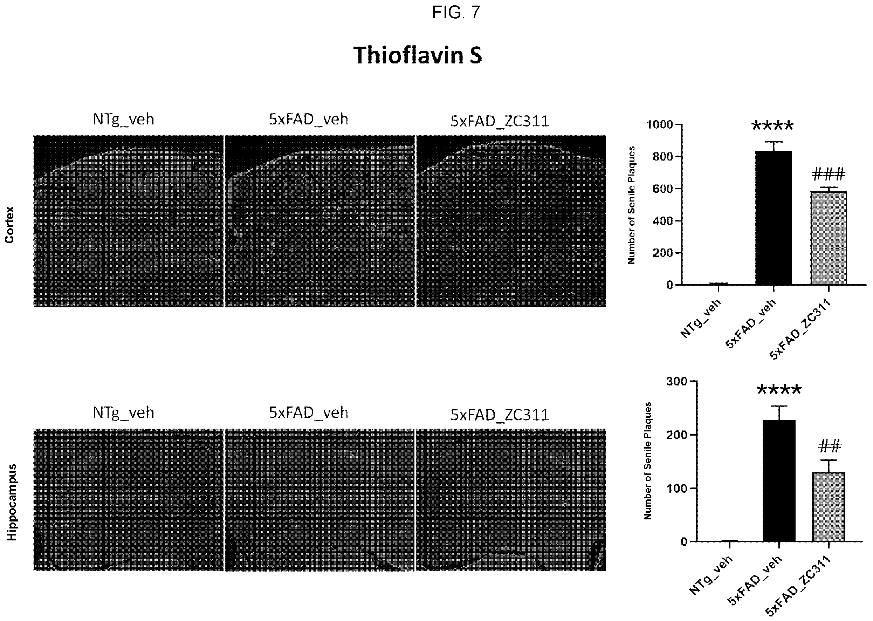

FIG. 7 is a series of fluorescence micrographs (left) and a graph (right) quantifying the results of thioflavin S staining of β-amyloid aggregate plaques in cortical (top) and hippocampal (bottom) slices from Alzheimer's disease model animals administrated with the peptides according to one embodiment of the present invention.

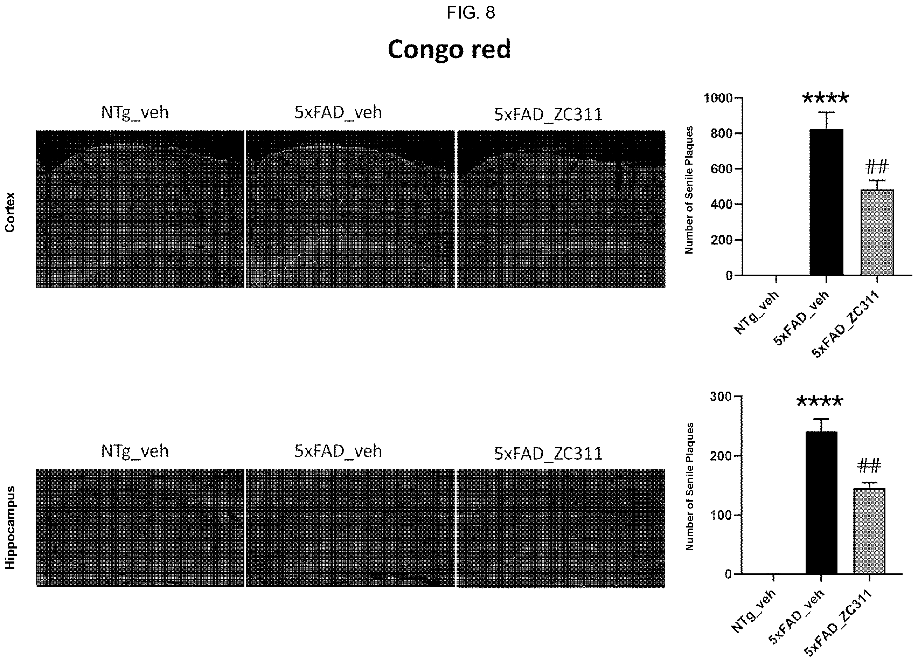

FIG.8 is a series of fluorescence micrographs (left) and a graph (right) showing the quantification of β-amyloid aggregate plaques stained with Congo red in cortical (top) and hippocampal (bottom) slices from the Alzheimer's disease model animals administrated with the peptides according to one embodiment of the present invention.

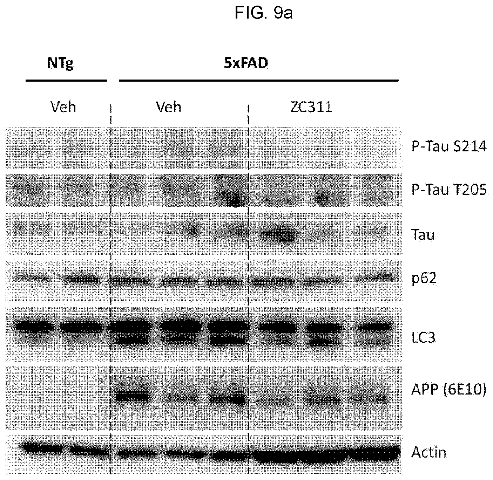

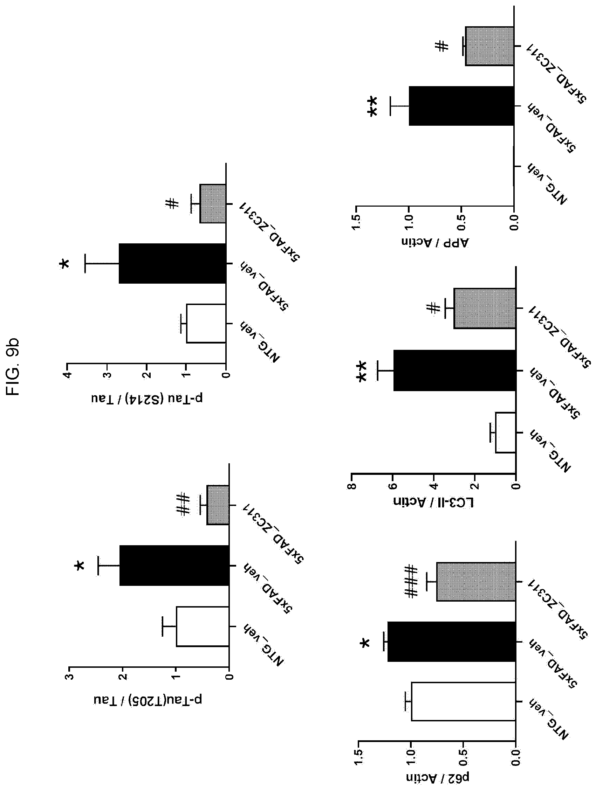

FIG.9a shows the results of a western blot analysis to determine the expression of various pathogenic aggregate-forming proteins in the cerebral cortex of the Alzheimer's disease model animals administrated with the peptides according to one embodiment of the present invention, and FIG.9b is a series of graphs quantifying the results of the Western blot analysis.

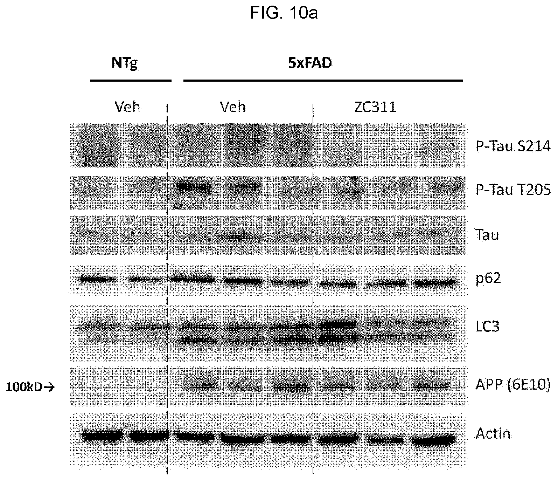

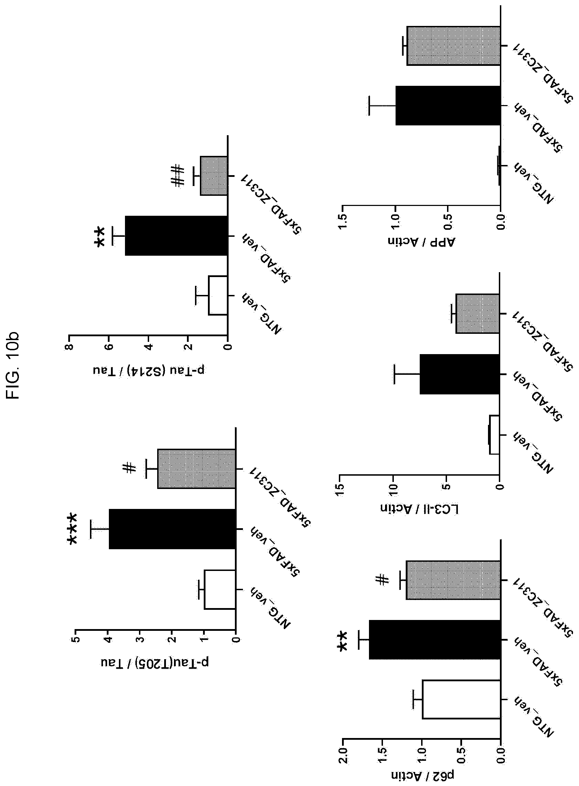

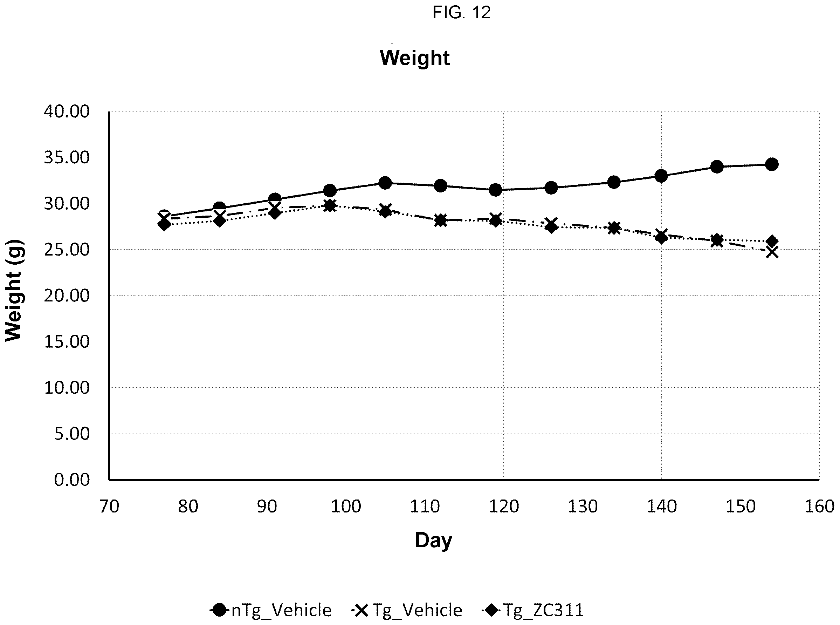

FIG. 10a shows the results of a western blot analysis to determine the expression of various pathogenic aggregate-forming proteins in the hippocampus of experimental animals administrated with the peptides according to one embodiment of the present invention, and FIG.12b is a series of graphs quantifying the results of the western blot analysis.

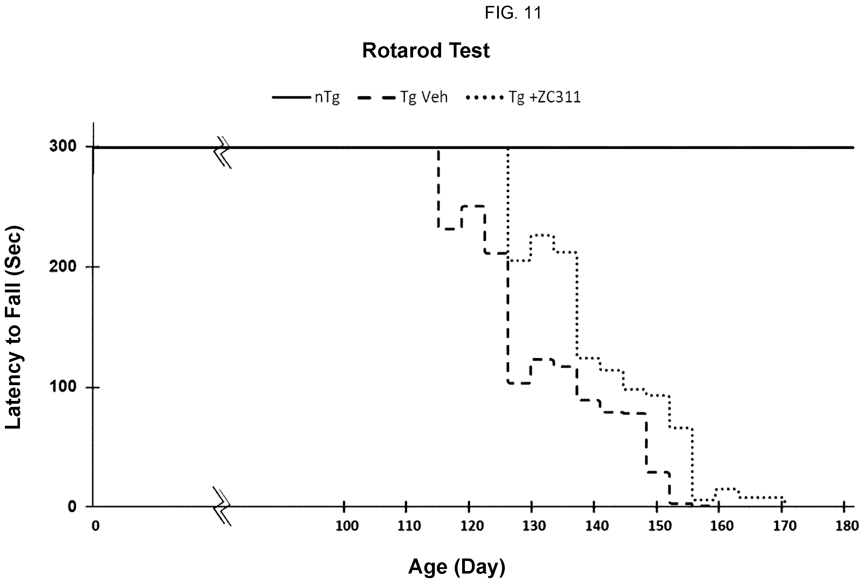

FIG. 11 is a graph depicting the results of a rotarod test analysis of motor function over time in ALS model animals treated with the peptides according to one embodiment of the present invention.
FIG. 12 is a graph representing the results of an analysis of changes in body weight over time in ALS model animals treated with the peptides according to one embodiment of the present invention.

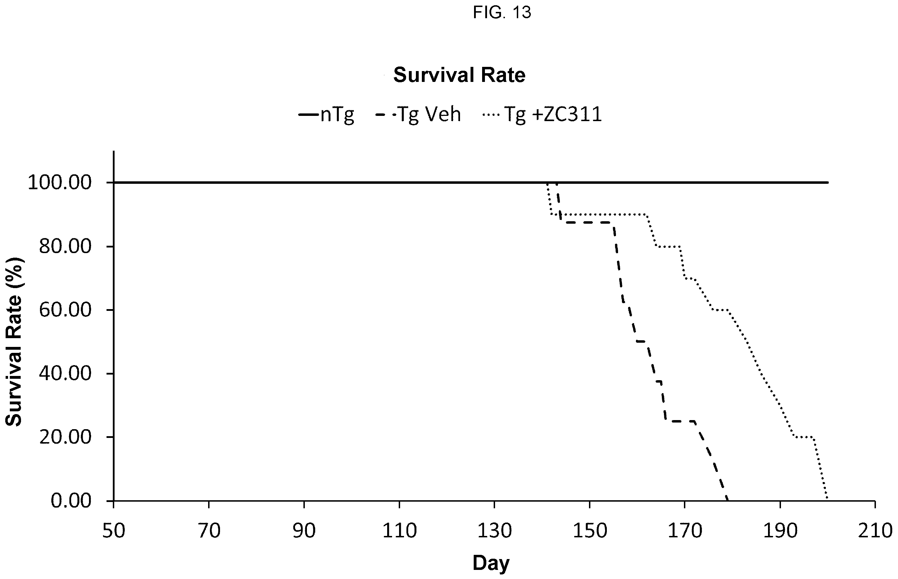

FIG. 13 is a graph depicting the results of analyzing survival over time in ALS model animals treated with the peptides according to one embodiment of the present invention.

### BEST MODE FOR THE INVENTION

### Definitions of terms:

As used herein, the term "zinc homeostasis" refers to the mechanism that maintains a constant level of intracellular zinc, which are known to involve zinc transporters, zinc-binding proteins (metallothioneins, MTs), and transcription factors (MTF1-2).

As used herein, the term "Neurodegenerative disease" refers to a disease characterized by the progressive loss of structure or function of nerves due to the abnormal death of nerve cells. These neurodegenerative diseases include amyotrophic lateral sclerosis (ALS), Parkinson's disease (PD), Alzheimer's disease (AD), and Huntington's disease (HD).

As used herein, the term "abnormal or pathological protein aggregates" refers to abnormal aggregation of proteins, such as amyloid or tau, within cells to form insoluble fibrills. These abnormal or pathological protein aggregates are known to be a neuropathologic hallmark of various intermittent or inherited neurodegenerative diseases.

### Detailed descriptions of the invention:

In one aspect of the present invention, there is provided a novel peptide with a length of from 4 to 10 a.a. and structural Formula 1 below, which has the ability to inhibit the formation of abnormal protein aggregates and autophagic activity against the protein aggregates.

H-G-X¹-X²-G-X³ (Structural formula 1)

(In the above structural formula, H is a L- or D-type histidine, G is glycine, X¹ is either absent or any D- or L-type amino acid, X² is any D- or L-type amino acid, and X³ is either absent or any D- or L-type amino acid except valine).

The peptide may comprise at least one D-amino acid.

In the peptide, X¹ may be absent or may be a non-polar or polar amino acid. The non-polar amino acid may be alanine, valine, leucine, methionine, isoleucine or proline, and the polar amino acid may be glutamine or asparagine. More preferably, the X¹ may be absent or may be D- or L-type valine or glutamine.

The peptide comprises the structure of Structural Formula 1, and may have a length of 4, 5, 6, 7, 8, 9 or 10 a.a.

In the peptide, the X² may be a polar amino acid, a non-polar amino acid, or an aromatic amino acid. The polar amino acid may be serine, cysteine, asparagine, glutamine, threonine or tyrosine, the non-polar amino acid may be alanine, valine, leucine, isoleucine, methionine or proline, and the aromatic amino acid may be tyrosine, tryptophan or phenylalanine. X² may more preferably be a D- or L-type amino acid selected from the group consisting of serine, histidine, valine, threonine, cysteine, and tryptophan.

In the peptide, the X³ may be a polar or non-polar amino acid, or an aromatic amino acid. The polar amino acid may be serine, cysteine, asparagine, glutamine, threonine or tyrosine, the non-polar amino acid may be alanine, valine, leucine, isoleucine, methionine or proline, and the aromatic amino acid may be tyrosine, tryptophan or phenylalanine. The X³ may more preferably be absent or may be a D- or L-amino acid selected from the group consisting of aspartic acid, serine, asparagine, tyrosine, histidine, and leucine.

Most preferably, the peptide may comprise or consist of an amino acid sequence selected from the group consisting of SEQ ID NOs: 1 to 15.

To investigate whether the production of abnormal protein aggregates that cause autophagy is inhibited by short peptides, the present inventors devised peptides in the form of various combinations of D- and L-amino acids as shown in Table 1 below, and then performed L3 puncta assays. As a result, as shown in FIGS. 1a to 1d, the peptides according to one embodiment of the present invention were found to promote autophagy by resolving the inhibition of autophagy induced by the bafilomycin treatment. These results demonstrate that the peptides according to one embodiment of the present invention can be used as a treatment for neurodegenerative diseases caused by abnormal protein aggregates by inhibiting the abnormal production of protein aggregates that accumulate in neurons and induce neuronal death, and by promoting autophagy and lysosomal activity against the abnormal protein aggregates produced.

In another aspect of the present invention, there is provided a pharmaceutical composition for the treatment of neurodegenerative diseases comprising the peptide as an active ingredient.

In the pharmaceutical composition, the neurodegenerative disease may be a neurodegenerative disease having the formation of abnormal protein aggregates as its etiology or pathology, wherein the abnormal protein aggregates are formed by the abnormal aggregation of α-synuclein, β-amyloid, TDP-43, p62 protein, FUS protein, superoxide dismutase-1 (SOD-1), huntingtin protein, or tau protein. The neurodegenerative disease may specifically be Alzheimer's disease (AD), which is associated with the accumulation of β-amyloid protein or tau protein aggregates; Parkinson's disease (PD), which is associated with the accumulation of α-synuclein aggregates; Amyotrophic lateral sclerosis (ALS), which is associated with the accumulation of SOD-1 aggregates; and Huntington's disease (HD), which is associated with the accumulation of Huntingtin protein aggregates; chronic traumatic encephalopathy, which is associated with the accumulation of aggregates of tau protein or TAR DNA-binding protein 43 (TDP-43); Lytico-bodig disease, which is associated with the accumulation of tau protein aggregates; fronto-temporal lobe degeneration, which is associated with the accumulation of tau protein, TAR DNA-binding protein 43 (TDP-43), fused in sarcoma (FUS) protein, or p62 protein aggregates; corticobasal degeneration, which is associated with the accumulation of tau protein aggregates; or progressive supranuclear palsy, which is associated with the accumulation of tau protein aggregates. Other diseases that have been associated with the accumulation of protein aggregates in the nerves include Creuzfeldt-Jakob disease, Gerstmann-Straussler-Scheinker syndrome, fatal familial insomnia, meningioangiomatosis, and neuronal ceroid lipofuscinoses. These neurodegenerative diseases and their association with the accumulation of protein aggregates in nerves are well described in the prior art (Strømland et al., J. Clin. Transl. Res. 2(1): 11-26, 2016; Tutar et al., Neurodegenerative Diseases, published: May 15th, 2013, DOI: 10.5772/54487; Diez-Ardanuy et al., Sci. Rep: 7(1): 10, 2017).

In the composition, the peptides can treat the neurodegenerative diseases by preventing neuronal cell death, regulating intracellular zinc homeostasis, and promoting lysosomal function.

The pharmaceutical composition according to one embodiment of the present invention may comprise a pharmaceutically acceptable carrier, and may further comprise pharmaceutically acceptable adjuvants, excipients or diluents in addition to the carrier.

As used herein, the term "pharmaceutically acceptable" refers to a composition that is physiologically acceptable and does not normally cause allergic reactions such as gastrointestinal disturbances, dizziness, or similar reactions when administered to humans. Experimental examples of such carriers, excipients, and diluents include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oils. It can further include fillers, anti-flocculants, lubricants, wetting agents, flavors, emulsifiers, and preservatives.

Furthermore, the pharmaceutical composition according to one embodiment of the present invention can be formulated using methods known in the art to allow for rapid release, or sustained or delayed release of the active ingredient upon administration to a mammal. Formulations include powders, granules, tablets, emulsions, syrups, aerosols, soft or hard gelatin capsules, sterile injectable solutions, and sterile powder forms.

The pharmaceutical composition according to one embodiment of the present invention can be administered by various routes, for example, orally, parenterally, e.g., by suppository, transdermally, intravenously, intra-abdominally, intra-peritoneally, intramuscularly, intralesionally, intranasally, intrathecally, or intrathecally. It can also be administered as a sustained release or by using an implantable device for continuous or repeated release. The number of doses may be administered once daily or divided into multiple doses within any desired range, and the duration of administration is not particularly limited.

The pharmaceutical composition according to one embodiment of the present invention can be administered by conventional systemic or localized administration, for example by intramuscular or intravenous injection. Furthermore, the peptides according to one embodiment of the present invention can also be administered orally.

The pharmaceutical compositions according to one embodiment of the present invention can be formulated in any suitable form with any commonly used pharmaceutically acceptable carrier. Pharmaceutically acceptable carriers include, for example, water, suitable oils, saline, and carriers for parenteral administration such as aqueous glucose and glycols, and may further include stabilizers and preservatives. Suitable stabilizers include antioxidants such as sodium hydrogen sulfite, sodium sulfite, or ascorbic acid. Suitable preservatives include benzalkonium chloride, methyl- or propyl-paraben, and chlorobutanol. The composition according to the present invention may also contain suspending agents, solubilizing agents, stabilizing agents, isotonic agents, preservatives, anti-adsorbents, surfactants, diluents, excipients, pH adjusters, painless relievers, buffering agents, and antioxidants, depending on the method of administration or formulation. Pharmaceutically acceptable carriers and formulations suitable for the present invention, including those exemplified above, are described in detail in the literature [Remington's Pharmaceutical Sciences, latest edition].

The dosage of the pharmaceutical composition according to one embodiment of the present invention to a patient depends on many factors, including the patient's height, body surface area, age, the specific compound being administered, sex, time and route of administration, general health, and other medications being administered concurrently. The therapeutically active peptide may be administered in an amount from 100 ng to 1000 mg per body weight (kg), more preferably in an amount of 1 µg to 100 mg per body weight (kg), and most preferably in an amount of 5 to 40 mg per body weight (kg), while the dose may be adjusted taking into account the above factors.

Furthermore, the pharmaceutical composition of the present invention are administered in a therapeutically effective amount.

As used herein, the term "therapeutically effective amount" means an amount sufficient to treat a condition with a reasonable benefit/risk ratio applicable to medical treatment, and the effective dose may be determined based on factors including the type and severity of the individual, age, sex, activity of the drug, sensitivity to the drug, time of administration, route of administration and rate of elimination, duration of treatment, concomitant medications, and other factors well known in the medical field. The pharmaceutical composition of the present invention may be administered at a dose of 0.1 mg/kg to 1 g/kg, more preferably at a dose of 1 mg/kg to 500 mg/kg. Meanwhile, the dosage may be appropriately adjusted according to the age, sex and condition of the patient.

In another aspect of the present invention, there is provided a method of treating a subject suffering from a neurodegenerative disease comprising administering the composition comprising a therapeutically effective amount of the peptide to the subject.

In another aspect of the present invention, there is provided a method of inhibiting the accumulation of pathogenic protein aggregates in the nervous system of a subject suffering from a neurodegenerative disease, comprising administering a therapeutically effective amount of the peptide to the subject.

In the above methods, the composition may be administered by oral or parenteral administration as described above, and in the case of parenteral administration, it may be administered by any of the routes of systemic or topical administration. In the case of systemic administration, it may be administered by intravenous injection (IV), intraperitoneal injection (IP), or intramuscular injection (IM). In the case of topical administration, it may be administered by intracranial administration, intrathecal administration, intracerebrospinal administration, subcutaneous injection (SC), and so on.

The method of any of the preceding claims, wherein the neurodegenerative disease may be a neurodegenerative disease having as an etiology or pathology the formation of abnormal protein aggregates, and the abnormal protein aggregates may be formed by the abnormal aggregation of α-synuclein, β-amyloid, TDP-43, p62 protein, FUS protein, superoxide dismutase-1 (SOD-1), huntingtin protein, or tau protein.

### MODE FOR THE INVENTION

The present invention will now be described in more detail with reference to the following examples. However, the present invention is not limited to the embodiments disclosed herein, but may be embodied in many different forms, and the following embodiments are provided to make the disclosure of the invention complete and to give those of ordinary skill in the art a complete idea of the scope of the invention.

### Example: Design and synthesis of peptides

The present inventors synthesized peptides with the amino acid sequences shown in Table 1 below, which were obtained from Anigen (Korea).

**Table 1**

| **Peptides svnthesized according to one embodiment of the present invention** | | | | | | | | |
|---|---|---|---|---|---|---|---|---|
| Peptides | | Amino acid sequences | | | | | | SEQ ID NO: |
| Example | Codename | H* | G | X¹ | X² | G | X³ | |
| 1 | ZC303 | H | G | v | s | G | -. | 1 |
| 2 | ZC304 | h | G | V | S | G | -. | 2 |
| 3 | ZC305 | h | G | v | s | G | d | 3 |
| 4 | ZC306 | h | G | v | s | G | s | 4 |
| 5 | ZC307 | h | G | v | s | G | n | 5 |
| 6 | ZC308 | h | G | v | s | G | y | 6 |
| 7 | ZC309 | h | G | V | s | G | h | 7 |
| 8 | ZC310 | h | G | v | S | G | h | 8 |
| 9 | ZC311 | h | G | Q | h | G | -. | 9 |
| 10 | ZC312 | h | G | q | v | G | -. | 10 |
| 11 | ZC313 | h | G | -. | v | G | -. | 11 |
| 12 | ZC314 | h | G | -. | h | G | L | 12 |
| 13 | ZC315 | h | G | v | t | G | -. | 13 |
| 14 | ZC316 | h | G | v | c | G | -. | 14 |
| 15 | ZC317 | h | G | v | w | G | -. | 15 |
| Uppercase letters represent L-type amino acids and lowercase letters represent D-type amino acids. | | | | | | | | |
| Those with * indicate can be either L- or D-type amino acid | | | | | | | | |
| Since glycine is not an optical isomer, there is no distinction between D- and L-types. (indicated with uppercase letters) | | | | | | | | |

### Experimental example 1: Cell culture

H4 cell line (GL-H4) and HEK293T cell line permanently transfected with GFP-LC3 plasmid were used in the present invention. The cell culture medium was Minimum Essential Medium (MEM, WellGene) supplemented with 10% fetal bovine serum (Hyclone, USA) and a mixture of antibiotic and antifungal agent (WellGene, Korea), and cultured in a cell culture machine at 37°C and 5% CO₂.

### Experimental example 2: Analysis of Autophagic Flow Blocked by Bafilomycin A-1

To investigate the effect of the peptides comprising the amino acid sequences designated as SEQ ID NOs: 1 to 15 on autophagic flow, the present inventors treated H4 cell lines expressing GFP-tagged LC3 (GL-H4) with MEM and then used the above peptides (20 µM) and the autophagy inhibitor Bafilomycin A-1, Baf A1, 100 nM) to inhibit autophagy. Later, the effect of the peptides according to one embodiment of the present invention was investigated by fluorescence microscopy analysis.

The LC3 puncta observed in FIGS. 1a and 1b are due to the presence of LC3-GFP proteins within autophagosomes. If the proteins are not degraded by lysosomes, the entire autophagic process is inhibited, leading to the accumulation of autophagosomes. Treatment of the GL-H4 cell line with 100 nM of bafilomycin (Baf A1) to inhibit the autophagic flow significantly increased the number and size of LC3 spots, which was reduced, albeit to varying degrees, upon treatment with the peptide (20 µM) according to one embodiment of the present invention (FIGS. 1a and 1b). Quantification by measuring the intensity of fluorescence of the whole spots also confirmed that the intensity of fluorescence was similarly reduced upon treatment with the peptides (ZC303 to ZC317) according to one embodiment of the invention (FIG.s 1c and 1d). This indicates that autophagosomes accumulated due to the blocked autophagy are scavenged by the peptides according to one embodiment of the present invention.

### Experimental example 3: Analyzing the effect of the novel peptide on protein aggregate formation after SOD1 protein overexpression

The present inventors conducted a Western blot analysis to investigate whether some of the peptides (ZC303, ZC304, ZC309, ZC311) comprising the amino acid sequences designated as SEQ ID NO: 1 to 15 could resolve SOD1 aggregates that formed after transient transfection of SOD1 protein into HEK293T cell lines. Specifically, the present inventors transiently transfected EGFP-SOD1 G93A DNA into the HEK293T cell line using Lipofectamine 2000. After transfection, the cell lines were incubated in a cell incubator at 37°C, 5% CO₂ for 30 h and the culture medium was replaced with Minimum Essential Medium (MEM, Gibco, USA). After a 30 min pre-treatment with 100 nM bafilomycin, 30 µM of peptide was added and incubated for 18 h of post-treatment. This was followed by lysis in Triton X-100 lysis buffer (30 mM HEPES pH 7. 5, 150 mM NaCl, 1% Triton X-100, 1 mN EDTA) supplemented with proteinase inhibitors and phosphatase inhibitors (2 µg/ml aprotinin, 2 µg/ml leupeptin, 1 µg/ml pepstatin A, 1 mM phenyl-methylsulfonyl fluoride (PMSF), 1 mM Na₃VO₄ , 5 mM NaF and 10 mM Na₄P₂O₇) in a 6-well culture dish at 200 µl per well, and the obtained cell lysate was left at 4°C for 30 min. The obtained cell lysate was subjected to protein quantification using BCA protein assay kit (Pierce Biotechnology, USA). Then, it was centrifuged at 17,000xg for 20 min, and only the supernatant was discarded, and the cell debris was taken to obtain protein extract. The protein extract was then resuspended in 5X sample buffer (300 mM Tris, pH 6.8, 10% SDS, 50% Glycerol, 0.1% Bromophenol blue, 2.5% Mercaptoethanol, 100 mM DTT) was mixed into the aliquoted sample and denatured at 95°C for 5 min to prepare the sample. Then, electrophoresis was performed using 8% to 15% SDS-polyacrylamide gel, and the proteins separated by their sizes were transferred to polyvinylidene difluoride (PVDF) membranes (Millipore, USA). The protein-transferred membrane was then blocked with 3% skim milk powder in TBST for 1 h. The blocked membrane was reacted with anti-GFP antibody (Santa Cruz biotechnology, USA), where anti-Actin antibody (Sigma, USA) was used as a loading control. The antibodies, each recognizing a specific protein, were added to a solution of 1% BSA dissolved in TBST. For all blots, secondary antibodies were reacted diluted to 1:10,000 in 2% skimmed milk powder. Enhanced chemiluminescence (iNTRoN Biotechnology, South Korea) and a bio-imaging system (MF-Chemibis, Shimadzu scientific Korea corporation, South Korea) were used to identify protein signals.

As a result, it can be seen that SOD1 protein aggregates have been increased by bafilomycin compared to the control, as shown in FIGS. 2a and 2b. Quantification also confirmed that the protein aggregates were reduced by some of the peptides according to one embodiment of the present invention, and the resolution of the aggregate accumulation was confirmed by some of the peptides according to one embodiment of the present invention.

### Experimental example 4: Analyze the effect of a novel peptide on protein aggregate formation after overexpression of mutant tau protein

The present inventors then investigated whether some of the peptides (ZC305, ZC306, ZC307, ZC311) comprising the amino acid sequences designated as SEQ ID NO: 1 to 15 could resolve the mutant tau protein aggregates that formed after transient transfection of the mutant tau protein into the HEK293T cell line. For this purpose, EGFP-Tau P301L DNA was transiently transfected into the HEK293T cell line using Lipofectamine 2000. Protein aggregate analysis was performed in the same manner as in Experimental example 3 above.

As a result, it can be seen in FIG. 3a that the mutant tau protein aggregates were increased by bafilomycin compared to the controls, and quantification also confirmed that the protein aggregates were reduced by some peptides according to one embodiment of the present invention.

### Experimental example 5: Analyzing the effect of a novel peptide on protein aggregate formation after overexpression of α-synuclein protein

The present inventors then investigated whether some of the peptides (ZC308, ZC310, ZC311, ZC312) comprising the amino acid sequences designated as SEQ ID NO: 1 to 15 could resolve the α-synuclein protein aggregates that formed after transient transfection of α-synuclein protein into HEK29T cell lines. For this purpose, EGFP-α-synuclein A53T DNA was transiently transfected into the HEK293T cell line using Lipofectamine 2000. Protein aggregate assays were performed in the same manner as in Experimental example 3 above.

As a result, it can be seen in FIG. 4 that α-synuclein protein aggregates have been increased by bafilomycin compared to the control, wherein protein aggregates were reduced by some peptides according to one embodiment of the present invention.

### Experimental example 6: Analyzing the effect of novel peptides on protein aggregate formation after overexpression of mutant huntingtin protein

The present inventors then investigated whether some of the peptides (ZC311, ZC314, ZC315, and ZC316) comprising the amino acid sequences designated as SEQ ID NO: 1 to 15 could resolve the mutant huntingtin protein aggregates that formed after transient transfection of the mutant huntingtin protein into the HEK293T cell line. For this purpose, GFP-mHttQ74 DNA was transiently transfected into the HEK293T cell line using Lipofectamine 2000. Protein aggregate analysis was performed in the same manner as in Experimental example 3 above.

As a result, it can be seen in FIG. 5 that mutant huntingtin protein aggregates have been increased by bafilomycin compared to the control, and that protein aggregates were reduced by some peptides according to one embodiment of the present invention.

### Experimental example 7: Alzheimer's model animal study

### 7-1: Analyzing change in body weights

Based on the results of Experiments 4 through 6 above, the present inventors conducted animal studies using disease model animals to determine whether the peptides according to one embodiment of the present invention can be used as therapeutics for various neurodegenerative diseases.

First, to determine whether *in vivo* administration of the peptide is toxic, dementia model animals (5XFAD) were administrated with 20 mg/kg of the peptide (ZC311) prepared in Example 9 of the present invention by intraperitoneal (IP) injection once daily, five days a week (Monday through Friday) for 25 weeks to determine changes in body weight. As controls, wild-type mice and Alzheimer's model mice were administrated with saline alone.

As a result, as shown in FIG. 6, weight gain was consistent over the 25-week injection period, with no differences between the experimental groups.

### 7-2: Analyze the Impact on β-Amyloid Aggregate Accumulation in the Brain

To determine whether the peptides according to one embodiment of the present invention could inhibit the accumulation of β-amyloid aggregates in the brain as a pathologic phenomenon of Alzheimer's disease, even under realistic *in vivo* conditions as shown by the results of Experiments 4 and 5 above, brain tissue sections obtained from experimental animals sacrificed after the peptide administration for 25 weeks were stained with thioflavin S and Congo red that have selective affinity for β-amyloid plaques.

Specifically, for thioflavin S staining, the brain of a dementia model mouse was harvested and freeze-dried in O.C.T compound (Sakura finetec, USA), and brain sections were obtained by coronal sectioning and mounted on glass slides coated with 0.1% poly-L-lysine. The obtained brain sections were washed in 70% ethanol for 1 min, then in 80% ethanol for 1 min again, and stained in 1% thiflavin S solution (MilliporeSigma, USA) for 15 min. After that, it was sequentially washed in 80% ethanol for 1 min, in 70% ethanol for 1 min again, and then washed twice in distilled water. Finally, the β-amyloid senile spots were observed through fluorescence microscopy.

Congo red staining to identify amyloid was performed as follows. Brain sections obtained by coronal sectioning were mounted on 0.1% poly-L-lysine coated glass slides. The obtained brain sections were washed in PBS (Phosphate-buffered Saline) solution for 2 min and then stained in 0.5% Congo red solution (Thermo fisher, USA) for 20 min. After washing the Congo red solution in PBS solution, the sections were rapidly dipped in and out of alkaline alcohol solution containing 0.01% sodium hydroxide 10 times. The sections were then washed in PBS solution for 3 min, followed by 95% ethanol for 3 min, and 100% ethanol for 3 min to dehydrate. The amyloid senile spots were observed under a fluorescence microscope, and the number of stained senile spots and amyloid per brain section was counted and quantified with a statistical program (Graph prism).

As a result, as shown in FIGS. 7 and 8, a significant number of β-amyloid plaques were identified in Alzheimer's model animals treated with PBS alone, but the accumulation of β-amyloid aggregates was significantly inhibited in Alzheimer's model animals treated with the peptide according to one embodiment of the present invention.

### 7-3: Analyzing the effects on the expression of different aggregation-forming proteins in the brain

Based on the results of Experimental example 7-2 above, the present inventors investigated the expression of pathogenic aggregate-forming proteins such as tau protein, p62, and LC3 in brain tissue other than β-amyloid by Western blot analysis.

Specifically, cortical and hippocampal tissues were obtained from experimental animals sacrificed after experiments, and the tissues were lysed and subjected to Western blot analysis in the same manner as Experimental example 3, using antibodies that specifically bind to phosphorylated tau S214 (P-Tau S214), phosphorylated tau T205 (P-Tau T205), unphosphorylated tau, p62, LC3, and β-amyloid (APP), respectively. As a result, as shown in FIGS. 9a to 10b, all six proteins in the cerebral cortex as well as in the hippocampus were increased in the Alzheimer's disease model mice compared to the control normal mice, whereas the animals treated with the peptide (ZC311) according to one embodiment of the present invention all showed significantly lower expression of the above aggregate-forming proteins in the cerebral cortex and hippocampus. These results demonstrate that the peptide according to one embodiment of the present invention can inhibit the formation of pathogenic protein aggregates in the brain even after *in vivo* administration.

### Experimental example 8: Confirming the effectiveness of a treatment for amyotrophic lateral sclerosis

### 8-1: Rotarod Test

To determine whether the peptide according to one embodiment of the present invention has therapeutic effects on amyotrophic lateral sclerosis (ALS) *in vivo,* the present inventors administered the peptide according to one embodiment of the present invention (ZC311) to an amyotrophic lateral sclerosis model animal (G93A-SOD1) at 40 mg/kg by subcutaneous (SC) injection repeatedly five days per week (Monday through Friday) from the age of 112 days to 200 days.

The animals were then subjected to a rotarod test twice a week at intervals of up to 3-4 days to evaluate their motor function. The rotarod test was performed using a rotarod treadmill (Cat. No. DJ-345, Dae Jong, Seoul, Korea) to evaluate impaired coordination, the main symptom of basal ganglia lesion disease, by measuring the time for the subject animals (n=11, 8, and 10) to fall off the rod during three repetitions of 300 seconds at a constant speed of 15 rpm. 300 seconds was set to be a perfect score, and the highest score was used.

As a result, as shown in FIG. 11, ALS model mice began to exhibit ataxia after 116 days and were completely ataxic by 150 days, whereas the development of ataxia was delayed in animals treated with the peptide (ZC311) according to one embodiment of the present invention.

### 8-2: Weight Change

The present inventors measured changes in body weight over time during the behavioral analysis of Experimental example 8-1 above.

As a result, as shown in FIG. 12, the ALS model animals progressively lost weight over time, a phenomenon that was not altered by administration of peptides according to embodiments of the present invention.

### 8-3: Survival Rate

Amyotrophic lateral sclerosis (ALS) is a devastating disease that progresses after onset and eventually leads to death. Animal models of ALS also eventually become 100% dead after a period of time after onset. The present inventors sought to investigate how the peptide according to one embodiment of the present invention affect survival in ALS model animals.

As shown in FIG. 13, the survival rate of animals undergoing the experiments in Experimental example 8-1 was measured over time, and it was found that the ALS model mice began to die at around 140 days, and by 178 days, all of the animals were dead (50% survival at 162 days). In contrast, ALS model mice treated with the peptide according to one embodiment of the present invention began to die at a similar time to the control group, but the time to death was delayed (50% survival 183 days). These results demonstrate that the peptide according to one embodiment of the present invention can delay ataxia, a key symptom of ALS, and subsequent death.

The present invention has been described with reference to the embodiments and experimental examples described above, but these are exemplary only, and one having ordinary skill in the art will understand that various modifications and equally other embodiments and experimental examples are possible from them. The true scope of technical protection of the invention should therefore be determined by the technical ideas of the appended claims of the patent.

### INDUSTRIAL APPLICABILITY

By inhibiting abnormal hyperphosphorylation of pathogenic proteins and the resulting aggregate formation in the central nervous system and removing parasitic protein aggregates, the peptides according to one embodiment of the present invention can be developed as therapeutic agents for various neurodegenerative diseases, such as Alzheimer's disease and amyotrophic lateral sclerosis, caused by pathogenic protein aggregates.

## Claims

1. A novel peptide with a length of from 4 to 10 a.a. and structural Formula 1 below, which has the ability to inhibit the formation of abnormal protein aggregates and autophagic activity against the protein aggregates.
H-G-X¹-X²-G-X³ (Structural formula 1)
(In the above structural formula, H is an L- or D-type histidine, G is glycine, X¹ is either absent or any D- or L-type amino acid, X² is any D- or L-type amino acid, and X³ is either absent or any D- or L-type amino acid except valine).

2. The peptide according to claim 1, wherein the peptide includes at least one D-type amino acid.

3. The peptide according to claim 1, wherein the X¹ is absent or is a nonpolar or polar amino acid.

4. The peptide according to claim 3, wherein the nonpolar amino acid is alanine, valine, leucine, methionine, isoleucine, or proline.

5. The peptide according to claim 3, wherein the polar amino acid is glutamine or asparagine.

6. The peptide according to claim 3, wherein the X¹ is absent or is valine or glutamine of D- or L-type.

7. The peptide according to claim 1, wherein the X² is a polar amino acid, a nonpolar amino acid, or an aromatic amino acid.

8. The peptide according to claim 7, wherein the polar amino acid is serine, cysteine, asparagine, glutamine, threonine or tyrosine.

9. The peptide according to claim 7, wherein the non-polar amino acid is alanine, valine, leucine, isoleucine, methionine or proline.

10. The peptide according to claim 7, wherein the aromatic amino acid is tyrosine, tryptophan, or phenylalanine.

11. The peptide according to claim 1, wherein the X² is a D- or L-type amino acid selected from the group consisting of serine, histidine, valine, threonine, cysteine, and tryptophan.

12. The peptide according to claim 1, wherein the X³ is absent or is a polar or nonpolar amino acid, or an amphiphilic amino acid.

13. The peptide according to claim 12, wherein the polar amino acid is serine, cysteine, asparagine, glutamine, threonine or tyrosine.

14. The peptide according to claim 12, wherein the nonpolar amino acid is alanine, valine, leucine, isoleucine, methionine or proline.

15. The peptide according to claim 12, wherein the aromatic amino acid is tyrosine, tryptophan, or phenylalanine.

16. The peptide according to claim 12, wherein the X³ is absent, or is a D- or L-amino acid selected from the group consisting of aspartic acid, serine, asparagine, tyrosine, histidine, and leucine.

17. The peptide according to claim 1, wherein the peptide comprises an amino acid sequence selected from the group consisting of SEQ ID NOs: 1 to 15.

18. A pharmaceutical composition for treating a neurodegenerative disease comprising the peptide of any one claim among claims 1 to 17 as an active ingredient.

19. The pharmaceutical composition according to claim 18, wherein the neurodegenerative disease has the formation of abnormal protein aggregates as its etiology or pathology.

20. The pharmaceutical composition according to claim 18, wherein the abnormal protein aggregates are formed by the abnormal aggregation of α-synuclein, β-amyloid, TDP-43, p62 protein, FUS protein, superoxide dismutase-1 (SOD-1), huntingtin protein, or tau protein.

21. The pharmaceutical composition according to claim 19, wherein the neurodegenerative disease is Alzheimer's disease (AD), Parkinson's disease (PD), Amyotrophic lateral sclerosis (ALS), Huntington's disease (HD), chronic traumatic encephalopathy (CTE), Lytico-bodig disease (LBD), frontotemporal lobar degeneration (FTD), Huntington's disease (HD), and chronic traumatic encephalopathy (CT), HD), chronic traumatic encephalopathy, Lytico-bodig disease, fronto-temporal lobe degeneration, corticobasal degeneration, or progressive supranuclear palsy.

22. A method of treating a subject suffering from a neurodegenerative disease, comprising administering to the subject a therapeutically effective amount of the peptide of any one claim among claims 1 to 17.

23. A method of inhibiting the accumulation of pathogenic protein aggregates in the nervous system of a subject suffering from a neurodegenerative disease, comprising administering to the subject a therapeutically effective amount of the peptide of any one claim among claims 1 to 17.
